Europäisches Patentamt

European Patent Office

Office européen des brevets

(11) Publication number: 0 348 969 B1

# EUROPEAN PATENT SPECIFICATION

(45) Date of publication of patent specification: **12.05.93**     (51) Int. Cl.[5]: **A61L 27/00, A61L 33/00**

(21) Application number: **89111839.0**

(22) Date of filing: **29.06.89**

(54) Method for rapid adherence of endothelial cells onto a surface and surfaces prepared thereby.

(30) Priority: **01.07.88 US 214240**

(43) Date of publication of application:
**03.01.90 Bulletin  90/01**

(45) Publication of the grant of the patent:
**12.05.93 Bulletin  93/19**

(84) Designated Contracting States:
**AT BE CH DE ES FR GB GR IT LI LU NL SE**

(56) References cited:
**EP-A- 0 124 200**
**EP-A- 0 186 084**
**US-A- 4 656 083**

**BIOMATERIALS, vol. 3, no. 2, April 1982,
pages 68-77, Butterworth & Co. (Publishers)
Ltd, Guildford, Surrey, GB; H. YASUDA et al.:
"Biomedical applications of plasma poly-
merization and plasma treatment of polymer
surfaces"**

(73) Proprietor: **Becton Dickinson and Company
One Becton Drive
Franklin Lakes New Jersey 07417-1880(US)**

(72) Inventor: **Williams, Joel L.
108 Beaver Pine Way
Cary North Carolina 27511(US)**
Inventor: **Montgomery, David B.
801 Brookgreen Drive
Cary North Carolina 27511(US)**
Inventor: **Baldwin, Lillian P.
15 Logging Trail
Durham North Carolina 27707(US)**
Inventor: **DiPisa, Joseph A.
84 Maryann Way
Wyckoff, NJ 07481(US)**

(74) Representative: **von Kreisler, Alek,
Dipl.-Chem. et al
Patentanwälte Von Kreisler-
Selting-Werner, Deichmannhaus am Haup-
tbahnhof
W-5000 Köln 1 (DE)**

BIOMATERIALS, vol. 8, no. 5, September 1987, pages 323–328, Butterworth & Co. (Publishers) Ltd, Guildford, Surrey, GB; P.B. VAN WACHEM et al.: "Adhesion of cultured human endothelial cells onto methacrylate polymers with varying surface wettability and charge"

TRANSACTIONS AMERICAN SOCIETY FOR ARTIFICIAL INTERNAL ORGANS, vol. 33, no. 3, September 1987, pages 631–635, Hagerstown, MD, US; K.L. BOYD et al.: "Endothelial cell seeding of ULTI carbon–coated small–diameter PTFE vascular grafts"

## Description

This invention relates to a method for attaching endothelial cells to a surface, and more specifically relates to a method for depositing a layer of endothelial cells on a polymeric surface and the surfaces prepared thereby.

Extensive investigations have been undertaken over many years to find materials that will be biologically and chemically stable toward body fluids. This area of research has become increasingly important with the development of various objects and articles which can be in contact with blood, such as artificial organs, vascular grafts, probes, cannulas, catheters and the like.

Synthetic plastics have come to the fore as preferred materials for such articles. However, such materials have the major drawback of being thrombogenic. Even such plastics as polytetrafluoroethylene and the silicone rubbers which are more compatible with blood than most plastics, still show thrombogenic characteristics.

Thrombogenicity has conventionally been counteracted by the use of anticoagulants such as heparin. Exemplary of heparinization procedures are the disclosures in U.S. − A − 4,613,517 and U.S. − A − 4,521,564.

Over the past three decades, artificial grafts have been used to restore blood flow to areas of ischemia, to provide blood flow for hemodialysis patients and for repair of arterial aneurysms. While these procedures are generally initially successful, long − term prognosis for patients receiving such grafts is not encouraging, principally because small diameter grafts (4mm or less) become occluded over time due to fibrin deposition and cellular adhesion due to the thrombogenic nature of the graft material.

The ideal blood − surface interface has long been considered to be the naturally occurring human endothelium, and much current research is focused on endothelialization procedures. Madri et al. in the Journal of Cell Biology 97, 153 (1983) reported that when cells are grown on interstitial collagens, they undergo proliferation and form a continuous cell layer. Williams et al., Journal of Surgical Research 38, 618 (1985) described pretreatment of prosthetic graft material with fibronectin, collagen or blood plasma, and reported that essentially no adherence occurred on untreated graft material, but that dramatic increases in adherence occurred on protein − coated polyester grafts. A similar study by Jarrell et al. (Annals of Surgery, 203, 671 (1986)) showed a high percentage of firm adherence of endothelial cells to polyester coated with platelet − rich − plasma in 10 min., to amnion/collagen coated polyester in 30 min. and to plain polyester in two hours, but that only the amnion/collagen coated surface exhibited complete graft coverage.

In recent years, attention has focused upon the poor results generally obtained with small diameter vascular grafts. van Wachem et al., in Biomaterials 6, 403 (1985) reported clinical success with polymeric grafts of greater than 4 mm, but that grafts of less than 4 mm gave generally disappointing clinical results due to immediate occlusion. Likewise, Baker et al., in American Journal of Surgery 150, 197 (1985) stated that long term patency of large diameter vascular grafts is relatively acceptable, but small diameter (less than 4 mm) grafts exhibit poor long − term patency rates.

Seeding of 4 mm inside diameter polyester vascular grafts with endothelial cells and patency after implantation in dogs is discussed by Belden et al. in Trans. Am. Soc. Artif. Intern. Organs. 28, 173 (1982).

Modification of polymeric surfaces by treatment with a variety of plasmas to achieve certain results is well known. For example, surface wettability, static properties and receptivity of a surface to deposition of a layer of an adherent polymeric material have been described. The doctoral thesis of Lee M. Smith, "Cell Adhesion As Influenced By Substrate Surface Properties", Department of Material Science and Engineering, the University of Utah, 1978, p. 67, suggests that cell adherence is a function of the carbon/oxygen ratio of the surface. van Wachem et al., (supra) discloses that endothelial cells can be cultured on glass or glow − discharge treated polystyrene.

U.S. − A − 4,452,679 discloses a method to modify a polymeric surface to introduce specific chemical groups by treatment of the surface with a plasma in which at least one of the neutral, positive or negative species of the plasma is excluded from contacting the surface.

Endothelial cells adhere partially, but not confluently, to an untreated Dacron® polyester surface, that this surface will become confluently covered in 24 hours, and that near confluent coverage occurs with a Dacron® surface pretreated with a protein, such as platelet rich plasma. In the present disclosure, the term confluent is used to describe a surface which is substantially covered with cells which are contiguous in all directions.

EP − A − 0 124 200 discloses the modification of polymeric surfaces by plasma treatment, whereby the plasma is generated from a gaseous material comprising, inter alia, nitrogen and/or oxygen.

The same is true for "Biomaterials" vol. 3, no. 2, April 1982.

From "Biomaterials", vol. 8, no. 5, September 1987 it is known that strong electrostatic interaction between the negatively charged cell − membrane and a positively charged surface occurs.

From Bruce E. Jarrell, Ann. Surg., June 1986, page 671 it is known that a confluent contact – inhibited monolayer of endothelial cells should have a density of $10^5$ endothelial cells per $cm^2$.

In spite of the extensive investigations on antithrombogenic prosthetic devices, the problem of thrombogenicity has not been satisfactorily solved, in particular with respect to small diameter grafts. It is toward the solution of this problem that the current invention is directed.

One aspect of the invention is a method to prepare a surface having a confluent layer of endothelial cells thereon according to claim 1. A polymeric substrate is exposed to a plasma generated from a material which includes nitrogen. The plasma treatment causes bonding of amino groups to the substrate. The substrate containing amino groups is contacted with endothelial cells which adhere to the substrate. The density of cells in the contacting medium is sufficient to cause the adhering cells forming a confluent layer on the substrate without cell proliferation. The substrate is polymeric.

Another aspect of the invention is an antithrombogenic article comprising a plasma – treated substrate having a confluent layer of adhered endothelial cells thereon according to claim 5. The articles are polymeric and may be of any shape. The preferred article is a conduit having an inside diameter of 0.5 mm or greater. One preferred, specific embodiment is a conduit having an inside diameter 2 to 6 mm. The lumen wall of these conduits is plasma – treated and endothelialized.

The plasma may be generated from any source of nitrogen which can be ionized. Preferred plasmas are generated from ammonia, most preferably ammonia containing a low concentration of oxygen.

The plasma treated substrate may be contacted with the endothelial cells in a suitable medium, such as a buffered saline, preferably under incubating conditions, to cause formation of an adhering confluent layer of the cells on the substrate.

Thus, in accordance with the invention articles having a plasma – treated polymeric surface and a confluent layer of adherent endothelial cells thereon are prepared by a two – step method which includes pretreatment of the article surface with a plasma to give an amine – rich surface and application of endothelial cells thereto. The plasma – treated surface prior to endothelialization is stable indefinitely and thus has a long shelf – life. Since the cells adhere quickly and become confluent without cell proliferation, a prosthetic device having the plasma – treated surface of the invention may be removed from stock, endothelialized, and be ready for implantation within minutes of procurement of sufficient cells. The advantages of speed to a patient undergoing surgery are apparent.

While this invention is satisfied by embodiments in many different forms, there will herein be described in detail preferred embodiments of the invention, with the understanding that the present disclosure is to be considered as exemplary of the principles of the invention and is not intended to limit the invention to the embodiments described.

One aspect of the present invention is a method for irreversibly modifying the surfaces of organic and inorganic substrates. More particularly, in accordance with the present invention, the surfaces of organic and inorganic substrates are irreversibly modified by bonding specific chemical functional species onto the surface of the substrate by contacting such surfaces with a plasma of a vaporized material in order to facilitate cell deposition and adherence.

The present invention can be employed to alter the surfaces of solid polymeric materials, including natural and synthetic addition and condensation polymers. Such polymeric materials include, but are not limited to, polyolefins, such as polyethylene, polypropylene, polyisobutylene and ethylene – alphaolefin copolymers, acrylic polymers and copolymers, such as polyacrylate, polymethylmethacrylate, polyethylacrylate; vinyl halide polymers and copolymers, such as polyvinyl chloride; polyvinyl ethers, such as polyvinyl methyl ether; polyvinylidene halides, such as polyvinylidene fluoride and polyvinylidene chloride; polyacrylonitrile, polyvinyl ketones; polyvinyl aromatics, such as polystyrene, polyvinyl esters, such as polyvinyl acetate; copolymers of vinyl monomers with each other and olefins, such as ethylene – methyl methacrylate copolymers, acrylonitrile – styrene copolymers, ABS resins, and ethylene – vinyl ace – tate copolymers; natural and synthetic rubbers, including butadiene – styrene copolymers, poly – isoprene, synthetic polyisoprene, polybutadiene, butadiene – acrylonitrile copolymers, polychloroprene rubbers, polyisobutylene rubber, ethylene – propylenediene rubbers, isobutylene – isoprene copolymers and poly – urethane rubbers; polyamides, such as Nylon 66 and polycaprolactam; polyesters such as polyethylene terephthalate, alkyd resins; phenol – formaldehyde resins; urea – formaldehyde resins, melamine – formal – dehyde resins, polycarbonates; polyoxymethylenes; polyimides; polyethers; epoxy resins, polyurethanes; wool; cotton; silk; rayon; rayon – triacetate; cellulose, cellulose acetate, cellulose butyrate; cellulose acetate – butyrate; cellophane; cellulose nitrate; cellulose propionate; cellulose ethers; and carboxymethyl cellulose.

The substrates may be any shape, relatively flat or curved, and may be of any composition, such as continuous or particulate, porous or impervious, and large or small. The invention can be employed for altering the surfaces of crystals, powders, plates, strips, films, sheets, wire, fibers, fabrics, filaments, tubing, and cast, extruded or compressed articles, and the like.

For most plasma treatments in accordance with the present invention, a conventional plasma generator may be used. Such generators may include thermal, radio frequency, direct current, audio frequency, and microwave plasmas using internal and external capacitive coupling, inductive coupling, resistive coupling, and waveguide techniques. Electrical excitation may be provided by means of a DC or low frequency AC glow discharge produced by internal electrodes or coupling using inductive or capacitive means with higher frequency power sources from audio frequencies up through radio frequency and into microwave frequencies. Microwave waveguide techniques may also be used. Exemplary of suitable plasma generators are the plasma reactors made by Branson/IPC and plasma surface treatment systems made by Plasma Science Inc.

For the preferred embodiment of the invention wherein a small diameter conduit is treated with plasma, an apparatus as disclosed in EP−A−0 348 690 may be used.

The plasma may be generated from any gas or gaseous mixture which provides reactive functional groups from the gas grafted onto the surface. Suitable gases are, for example, oxygen, nitrogen and low molecular weight organic or inorganic compounds of oxygen or nitrogen such as methanol, acetonitrile or hydrogen cyanide. Preferred gases are nitrogenous such as nitrogen, aliphatic amines of from 1 to 6 carbon atoms, hydrazine, ammonia or mixtures thereof. Other components may be present in the gas mixture such as are hydrogen, halogen and inert gases such as helium, oxygen, neon, krypton and xenon.

The preferred plasma for surface modification in preparation for cell adherence is generated from ammonia containing a low concentration of oxygen. The oxygen to ammonia ratio may be from about 0.005 to 0.8, preferably from about 0.01 to 0.1. The desired ratio may be achieved by bleeding appropriate quantities of the gases into the reaction chamber after conventional pumpdown. However, for most surface treatments in accordance with the present invention, sufficient oxygen remains in the vacuum chamber after pumpdown to achieve the desired ratio. It is evident that the quantity of oxygen remaining in the chamber is related to the pumpdown pressure in the evacuated chamber. Thus a desired oxygen−nitrogen ratio within an appropriate total gas pressure may be obtained by evacuating the chamber to a pre−determined pressure and bleeding in the required amount of ammonia. In general, a close approximation of the desired oxygen concentration can be achieved merely by control of the pumpdown time.

Plasma generation for surface modification in accordance with the present invention may be carried out with a wide range of power settings, radio frequencies, durations of exposure, temperatures, and gas pressures. Ranges for these parameters which provide advantageous results are measured DC or AC power density levels of from 0.001 to 400 watts per cubic centimeter, oscillation frequencies up to 100 megahertz, 2 seconds to 12 hours, 0 to 200° C, and $1.3 \times 10^{-5}$ to $1.3 \times 10^{-1}$ bar (0.01 to 100 torr). Preferred ranges for these parameters are .01 to 200 watts per cubic centimeter, 5 to 30 megahertz, 5 seconds to 120 minutes, 10−50° C, and $1.3 \times 10^{-5}$ to $2.6 \times 10^{-2}$ bar (0.01 to 20 torr). Gas flow rates may vary from stagnant conditions to several volume replacements per second.

The pumpdown pressure which controls the oxygen concentration may be from about 0.1 to 100, preferably, about $6.6 \times 10^{-6}$ to $6.6 \times 10^{-5}$ bar (5 to 50 millitorr). Depending on the capacity of the pump, these pumpdown pressures may be reached in about 1 min. to 72 hours.

The polymeric surface of the invention modified by exposure to a plasma generated from an ammonia−oxygen mixture contains amino groups bonded onto its surface. In addition, oxygen containing groups, such as carbonyl, carboxyl, hydroxyl, ether and peroxide groups are present consequent to reaction of the surface with reactive oxygen species.

In the following examples, polymeric substrates are subjected to a plasma generated from an oxygen−ammonia mixture. The plasma−treated substrate surfaces may be examined by conventional electron spectroscopy for chemical analysis (ESCA), to measure binding energy shifts and determine therefrom the nature of the functional groups on the surface. Comparative ESCA data for the plasma−modified surfaces of the invention and untreated surfaces are given in Table I.

The amino and oxygen−containing groups introduced onto the substrate surface by the plasma render the surface amenable to the deposition and adherence of cells. In the second step of the method of the invention, endothelial cell types may be applied to the plasma treated surfaces of the invention. Methods to isolate and purify endothelial cells from various sources are well known, and the provision of cells for adherence to the plasma−treated substrate surface of the invention is not part of the invention.

Endothelialization may be carried out by contacting the plasma−treated substrate surface with sufficient cells suspended in a medium to cause formation of an adhered confluent layer without a requirement for cell proliferation. In accordance with the invention, this technique and the suspending medium are referred to as sodding and the sodding medium respectively.

Any sodding medium which is not deleterious to the cells may be used. Preferred sodding media are buffers, as for example, buffered saline. The cells adhere confluently in a manner of minutes, generally in about 1 to 60 minutes. If desired, adherence may be hastened by incubating the sodded surface at a temperature of from about 10 to 50° C, preferably at 37° C.

The number of cells required to sod the plasma‒treated surface varies somewhat in accordance with the particular polymer and the particular surface chemistry after plasma treatment. In general about $10^5$ cells per square centimeter of surface to be covered is sufficient, but a greater concentration may also be used. Determination of this number and the concentration of cells in the sodding medium, which is not critical, are well within the purview of one skilled in the art. Likewise, determination of cell adherence and confluency by scanning electron microscopy or light microscopy after staining are conventional.

In another aspect of the invention, antithrombogenic articles having a plasma‒treated surface and a confluent layer of endothelial cells adhered directly to the plasma‒treated surface are provided. Exemplary of articles contemplated to fall within the scope of the invention are prosthetic devices which come into contact with blood, and include, as non‒limiting examples, artificial hearts, heart valves, pins and preferably, vascular grafts. The most preferred article of the invention is a small diameter vascular graft, such as a conduit of inside diameter of 6 mm or less having a plasma‒treated and endothelialized lumen wall.

Example I

Flat polyurethane film samples were modified in an RF plasma discharge system. A pair of 20.3 cm (8 inch) diameter aluminum electrodes spaced 9.5 cm (3 3/4 inches) apart were used inside a 30.5 cm (12 inch) diameter 55.8 cm (22 inch) tall vacuum chamber. RF power was delivered from a variable frequency oscillator/amplifier pair into a "T" matching network, through a balun transformer into the vacuum chamber using a sealed feedthrough, and then the balanced lines were connected to the opposing horizontal electrodes. The film samples were placed atop the lower electrode.

The chamber was pumped down to 60 mTorr over a period of 6 minutes. While continuing to pump, anhydrous ammonia gas was bled through a fine metering valve into the chamber at a rate sufficient to maintain a $2.6 \times 10^{-1}$ bar (200 mTorr) pressure. After thus purging the system for 1 minute, a 10.5 MHz 25 watt RF plasma was produced for 2 minutes to treat the film. Following treatment, the system was vented to atmosphere and the samples removed.

The surface chemistry of the samples was measured using ESCA and the resulting elemental compositions for treated and untreated samples are given in Table I.

Table I

| Elemental Atomic Percent Values for Plasma Treated and Virgin Polyurethanes | | | | | | |
|---|---|---|---|---|---|---|
| Substrate | Plasma Treated | | | Virgin Polymer | | |
| | Carbon | Oxygen | Nitrogen | Carbon | Oxygen | Nitrogen |
| Polyurethane I | 71 | 18 | 7 | 83 | 12 | 3.9 |
| Polyurethane II | 77 | 12 | 9.3 | 91 | 4.5 | 4.2 |
| Polyurethane III | 72 | 18 | 7.7 | 74 | 20 | 5.1 |
| Polyurethane IV | 67 | 19 | 11 | 76 | 20 | 3.9 |
| Polyurethane V | 72 | 14 | 7.9 | 86 | 8.2 | 3.6 |

Example II

Plasma Treatment of a Small Diameter Polystyrene Conduit

The inside wall of a section of polystyrene tubing 15 cm long and a 2.5 mm inside diameter was subjected to a plasma generated in the plasma generator of EP‒A‒0 348 690. Conditions of plasma treatment were similar to those used in Example I except that a 11.4 MHz 75 watt RF plasma was used and ammonia gas pressure was maintained at $1.86 \times 10^{-2}$ bar (14 Torr). Treatment duration was 25 seconds.

Example III

Plasma Treatment of a Small Diameter Polyurethane Conduit

The inside wall of a section of polyurethane conduit 100 cm long and 3.5 mm inside diameter was plasma modified as described in Example II.

Example IV

General Procedure for Deposition of Endothelial Cells onto a Polymer Surface

A plasma treated polymer film was cut into disks having an area of 2 $cm^2$, and the disks were placed into the wells of a FALCON® 24 – well tissue culture plate. A series of experimental controls was also prepared by adding one milliliter of 1% gelatin to each of six wells and one ml of 0.9% saline to six other wells. One ml of 0.9% saline was added to each well containing a plasma treated polymer disk. Both 24 – well plates were covered with parafilm and maintained at 4°C for 24 hours to ensure adequate hydration of test and control surfaces.

The gelatin and saline solutions were removed by aspiration and 0.8 ml of a stock suspension containing 2.5 x $10^5$ endothelial cells per ml was added to the wells. The cells were incubated for one hour at 37°C in a 5% carbon dioxide atmosphere, then washed with buffer and decanted using trypsin. The test surfaces and controls were washed again and the adherent cells were stained with hematoxylin. The number of adherent cells was determined using standard light microscopy techniques.

The results of this experiment are given in Table II as adherent endothelial cell population density, in cells per $cm^2$ for a variety of plasma treated polymer formulations.

EP 0 348 969 B1

Table II

Endothelial Cell Adherence to
Plasma Treated Polymer Disks

| Polymer* | Adherent Cell Density (cells/cm$^2$) |
|---|---|
| Polyethylene Terephthalate | |
| sample PET-1 | 15800 |
| sample PET-2 | 31000 |
| Polystyrene | |
| sample PS-1 | 44200 |
| sample PS-2 | 30200 |
| Polyurethane | |
| sample PU-1 | 25200 |
| sample PU-2 | 35700 |
| sample PU-3 | 46800 |
| sample PU-4 | 34500 |
| sample PU-5 | 30700 |
| Polyolefin | |
| sample PO-1 | 19700 |
| sample PO-2 | 60500 |
| sample PO-3 | 64800 |
| sample PO-4 | 14200 |
| Saline Control | 12600 |
| Gelatin Control | 40100 |

*Each polymer sample represents a different polymer formulation.

8

Example V

Effect of Plasma Treatment of the Adherence of Primary Endothelial Cells

Following the general procedure for deposition of endothelial cells onto a polymer surface described in Example IV, an inoculum of primary human endothelial cells was applied to plasma treated and virgin polyurethane disk samples. Two polyurethane types were examined, with cell adherence results shown in Table III. While primary endothelial cells do not exhibit the same propensity for attachment to polymer surfaces as cultured endothelial cells, the relative affinity for plasma treated polymer surfaces is higher than for untreated virgin polymer surfaces.

Table III

| Primary Endothelial Cell Adherence to Plasma Treated and Virgin Polyurethane | |
|---|---|
| Polymer | Adherent Cell Density (cells/cm$^2$) mean ± standard deviation |
| Polyurethane A | |
| plasma treated<br>untreated virgin | 14606 ± 7211<br>6287 ± 2183 |
| Polyurethane B | |
| plasma treated<br>untreated virgin | 15058 ± 7607<br>7005 ± 5301 |

Example VI

Deposition of Cells Onto the Inside Wall of a Plasma

Treated Small Diameter Polyurethane Conduit

Polyurethane conduits of Example III with internal diameter of 3.5 mm were plasma treated using the conditions described in Examples I and II. A primary endothelial cell population was suspended in buffer as described in Example IV at an inoculation density of $2 \times 10^5$ endothelial cells per square centimeter of polymer surface area calculated for the internal lumen of the conduits.

Each tube was encased in a supporting glass tube with connecting ports into which the cell suspension and buffer were introduced. The tubes were divided into two groups for incubation. Group I was rotated axially at a constant rate of 360 per minute and Group II was rotated axially in increments of 90 every fifteen minutes. Both groups were incubated for one hour at 37°C in a 5% carbon dioxide atmosphere. A gelatin control was also prepared according to the procedure of Example IV.

The cell adherence results appear in Table IV. Both methods of incubation allow endothelial cell adherence, however, a more uniform layer is achieved using the constant rotational method.

Table IV

| Endothelial Cell Adherence to Plasma Treated Polyurethane Conduits | | |
|---|---|---|
| Conduit Sample Number | Adherent Cell Density (cells/cm$^2$) | |
| | Group I (constant rotation) | Group II (incremental rotation) |
| Sample 1 | 20753 | 22429 |
| Sample 2 | 34539 | 8211 |
| Sample 3 | 14595 | 19397 |
| Sample 4 | 21017 | 10414 |
| Gelatin Control = 14652 | | |

**Claims**

1. A method for the endothelialization of a polymeric surface by
    a) contacting the surface with a plasma generated from a gas which includes nitrogen to provide reactive functional amino groups from said gas to said surface, characterized by
    b) applying to said surface endothelial cells by sodding a confluent layer of cells without a requirement for cell proliferation.

2. The method in accordance with claim 1 wherein said gaseous material further comprises a second material selected from the group consisting of hydrogen, halogen, argon, neon, krypton and xenon, oxygen and a compound of oxygen.

3. The method in accordance with claim 1 comprising treating a lumen wall of a polymeric conduit and sodding said lumen wall with endothelial cells.

4. The method of claim 3 wherein the lumen wall to be treated has an inside diameter of 4 mm or less.

5. An antithrombogenic article comprising a plasma – treated polymeric surface having reactive functional amino groups bonded thereto and a confluent layer of endothelial cells without the requirement of cell proliferation adhered to said surface containing reactive functional amino groups.

6. The article in accordance with claim 5 selected from the group consisting of a plate, strip, film, sheet, fiber, fabric, conduit and cast.

7. The article in accordance with claim 5 wherein said functional groups further comprise groups containing oxygen.

8. The article in accordance with claims 5 to 7 having an inside diameter less than about 6 mm comprising a plasma – treated lumen wall.

**Patentansprüche**

1. Verfahren zur Endothelialisierung einer Polymeroberfläche durch
    (a) In – Berührung – Bringen der Oberfläche mit einem Plasma, das aus einem Gas, das Stickstoff enthält, erzeugt wurde, um reaktionsfähige funktionelle Aminogruppen aus dem Gas für die Ober – fläche zur Verfügung zu stellen, dadurch gekennzeichnet, daß
    (b) an der Oberfläche Endothelzellen durch Aufschwemmen einer zusammenfließenden Zellschicht ohne Erfordernis der starken Zellvermehrung angebracht wurden.

2. Verfahren gemäß Anspruch 1, worin das gasförmige Material weiterhin ein zweites Material umfaßt, das aus der aus Wasserstoff, Halogen, Argon, Neon, Krypton und Xenon, Sauerstoff und einer Sauerstoff – verbindung bestehenden Gruppe ausgewählt ist.

3. Verfahren gemäß Anspruch 1, umfassend die Behandlung einer Lumenwandung einer Polymerleitung und das Aufschwemmen der Lumenwandung mit Endothelzellen.

4. Verfahren nach Anspruch 3, worin die zu behandelnde Lumenwandung einen Innendurchmesser von 4 mm oder weniger hat.

5. Antithrombogener Gegenstand, umfassend eine Plasma – behandelte Polymeroberfläche mit reak – tionsfähigen funktionellen, an dieselbe gebundenen Aminogruppen und eine zusammenfließende Schicht von Endothelzellen ohne Erfordernis der starken Zellvermehrung, die an die die reaktionsfähi – gen funktionellen Aminogruppen enthaltende Oberfläche geklebt ist.

6. Gegenstand gemäß Anspruch 5, der aus der Gruppe, bestehend aus einer Platte, einem Streifen, einem Film, einer Folie, einer Faser, einem Gewebe, einer Leitung und einer Form ausgewählt ist.

**7.** Gegenstand gemäß Anspruch 5, worin die funktionellen Gruppen des weiteren Sauerstoff enthaltende Gruppen umfassen.

**8.** Gegenstand gemäß Anspruch 5 bis 7, der einen Innendurchmesser von weniger als etwa 6 mm hat, umfassend eine Plasma – behandelte Lumenwanandung.

**Revendications**

**1.** Procédé pour endothélialiser une surface polymère, consistant :
a) à mettre en contact la surface avec un plasma produit par un gaz comprenant de l'azote, de façon à conférer à ladite surface des groupes amino fonctionnels réactifs provenant dudit gaz, caractérisé en ce qu'il consiste
b) à appliquer des cellules endothéliales sur ladite surface, en plaçant par – dessus une couche confluente de cellules sans nécessiter de prolifération cellulaire.

**2.** Procédé selon la revendication 1, dans lequel ledit matériau gazeux comprend en outre un deuxième matériau choisi parmi l'ensemble comprenant l'hydrogène, les halogènes, l'argon, le néon, le krypton et le xénon, l'oxygène et un composé de l'oxygène.

**3.** Procédé selon la revendication 1, qui consiste à traiter une paroi intérieure d'un conduit polymère et à recouvrir cette paroi intérieure de cellules endothéliales.

**4.** Procédé selon la revendication 3, dans lequel la paroi intérieure à traiter possède un diamètre intérieur de 4 mm ou moins.

**5.** Article antithrombogène, comprenant une surface polymère traitée par un plasma, à laquelle sont fixés des groupes amino fonetionnels réactifs et une couche confluente de cellules endothéliales sans nécessiter de proliférations cellulaires, qui adhèrent à ladite surface contenant des groupes amino fonctionnels réactifs.

**6.** Article selon la revendication 5, choisi parmi l'ensemble comprenant les plaques, les bandes, les films, les feuilles, les fibres, les tissus, les conduits et les objets moulés.

**7.** Article selon la revendication 5, dans lequel lesdits groupes fonctionnels comprennent en outre des groupes contenant de l'oxygène.

**8.** Article selon les revendications 5 à 7, ayant un diamètre intérieur inférieur à environ 6 mm et comprenant une paroi intérieure traitée par un plasma.